# EUROPEAN PATENT APPLICATION

(11) **EP 2 517 691 A2**
(43) Date of publication of application: **31.10.2012**
(21) Application number: 12162683.2
(22) Date of filing: 21.12.2006
(51) Int. Cl.: A61K 8/25, A61Q 11/00, A61K 8/34

(54) **Abrasive system for oral care compositions**

(30) Priority: 21.12.2005 US 752340 P
(62) Divisional of application: 06850354.9
(71) Applicant: Colgate-Palmolive Company, New York, New York 10022 (US)
(72) Inventor: Prencipe, Michael, Princeton Junction, NJ New Jersey 08550 (US); Ibrahim, Sayed, Somerset, NJ New Jersey 08873 (US)
(74) Representative: Jenkins, Peter David

(57) **Abstract**

Oral compositions comprising a first and a second abrasive are provided. The first abrasive preferably has an Einlehner hardness of greater than about 5 mg loss per 100,000 revolutions, and the second abrasive preferably has an Einlehner hardness of less than about 5 mg loss per 100,000 revolutions. A ratio of the first abrasive to the second abrasive ranges from about 1:1.6 to about 1.6:1, and the first and second abrasives are preferably present in the oral composition at an amount of about 13 % to about 21% by weight, respectively. The pellicle cleaning ratio of the oral composition is greater than 100 and the radioactive dentin abrasiveness is preferably less than 200, and in certain embodiments, preferably less than about 175. Methods of using the oral compositions are also provided.

## Description

### CROSS REFERENCE TO RELATED APPLICATION

The present application claims benefit of United States Provisional Application No. 60/ 752,340 filed December 21, 2005, which is hereby incorporated by reference in its entirety.

### BACKGROUND OF THE INVENTION

Abrasives in oral compositions debride and physically scrub the external surface of the teeth. This scrubbing action removes organic biofilm (i.e., the pellicle) on the tooth surface that is formed primarily of salivary proteins, bacteria, and bacterial byproducts. It can be stained and discolored by foods, such as coffee, tea and berries, as well as, by tobacco smoke, cationic compounds, and chromogenic bacteria. Such physical removal of the stained pellicle is a simple and effective means of removing the undesirable surface staining and discoloration which occurs daily. Further, such physical removal of the pellicle also removes plaque bacteria on the pellicle surface, thereby minimizing the potential for gingivitis, periodontitis, and caries formation. However, oral compositions such as dentifrices should not have such high abrasiveness that potential damage to the enamel or tissue may result. As such, it is desirable to develop oral compositions that optimize the cleaning and/or polishing efficacy of the oral composition, while minimizing the harmful abrasiveness to avoid potential damage oral surfaces.

### BRIEF SUMMARY OF THE INVENTION

In various embodiments, the present invention is directed to an oral composition comprising:
a first abrasive having an Einlehner hardness of greater than about 5 mg loss per 100,000 revolutions; and
a second abrasive having an Einlehner hardness of less than about 5 mg loss per 100,000 revolutions;
wherein the ratio of the first abrasive to the second abrasive is about 1:1.6 to about 1.6:1, and wherein oral composition has a pellicle cleaning ratio (PCR) of greater than about 100 and a radioactive dentin abrasion (RDA) of less than about 200.

In various embodiments, the present invention is directed to an oral composition comprising:
a first abrasive comprising silica, having an Einlehner hardness of greater than about 5 mg loss per 100,000 revolutions and an oil of absorption of less than about 90 cm³/100g; and
a second abrasive comprising silica, having an Einlehner hardness of less than about 5 mg loss per 100,000 revolutions and an oil of absorption of greater than about 90 cm³/100g;
wherein the first abrasive is present in an amount of about 13% to about 21 % by weight and the second abrasive is present in an amount of about 13% to about 21% by weight of the composition.

In various embodiments, the present invention is directed to an oral composition comprising:
a first abrasive comprising silica and having an oil of absorption of less than about 90 cm³/100g and an Einlehner hardness of greater than about 5 mg loss per 100,000 revolutions; and
a second abrasive comprising silica and having an oil of absorption of greater than about 90 cm³/100g and an Einlehner hardness of less than about 5 mg loss per 100,000 revolutions;
wherein a ratio of the first abrasive to the second abrasive is about 1:1.6 to about 1.6:1, and a total amount of the first and second abrasives present in the oral composition is greater than about 25% by weight of the composition; and wherein the oral composition has a pellicle cleaning ratio of greater than about 100 and a radioactive dentin abrasion of less than about 200.

In various embodiments, the present invention provides methods of increasing delivery of an active ingredient in an oral composition to an oral surface comprising:
introducing an oral composition into an oral cavity, wherein the oral composition comprises the active ingredient, a first abrasive and a second abrasive, wherein the first and second abrasives each comprise silica and each are present in an amount of about 13 % to about 21 % by weight, wherein the oral composition has a pellicle cleaning ratio of greater than about 100 and a radioactive dentin abrasion of less than about 200. The composition is contacted with the oral surface. The delivery of the active ingredient in the oral composition is greater than a comparative composition having an abrasive comprising silica and a pellicle cleaning ratio of less than about 100; and
contacting the composition with the oral surface, wherein the delivery of the active ingredient is greater than a comparative composition having an abrasive comprising silica and a pellicle cleaning ratio of less than about 100.

In various embodiments, the present invention is directed to an oral care composition comprising:
a first abrasive having an Einlehner hardness of greater than about 10 mg loss per 100,000 revolutions; and
a second abrasive having an Einlehner hardness of less than about 10 mg loss per 100,000 revolutions;
wherein the ratio of the first abrasive to the second abrasive is about 1:1.6 to about 1.6:1, and wherein oral composition has a pellicle cleaning ratio (PCR) of greater than about 100 and a radioactive dentin abrasion (RDA) of less than about 200.

### DETAILED DESCRIPTION OF THE INVENTION

As used herein, all measurement levels described herein are by weight of the total composition, unless otherwise indicated. Additionally, all references cited herein are hereby incorporated by reference in their entireties. However, in the event of a conflict between any definitions in the present disclosure and those in a cited reference, the present disclosure controls.

In various embodiments, an oral composition is provided that has a first abrasive and a second abrasive. Abrasive selection for the present oral compositions may account for the abrasive type, fineness (particle size), particle size distribution and amount of abrasive, to ensure that tooth enamel is not excessively abraded during normal use of the composition, but is sufficiently cleaned and/or polished. In the context of oral care, the efficacy of the abrasive can be expressed based on a cleaning or an abrasion basis for a dentifrice, namely the pellicle cleaning ratio (PCR) or the radioactive dentin abrasion (RDA) respectively. Methods of performing PCR and RDA are described in e.g., United States Patent Nos. 5,939,051 and 6,290,933.

In commonly accepted conventional practice, RDA values for an oral composition are generally kept below 250 to avoid harming enamel/ dentin with repeated usage. However, in order to achieve a higher PCR, typically the amount and hardness of abrasives must be increased, which is conventionally known to increase the RDA. For example, it has been observed that when the RDA value of a dentifrice composition exceeds certain values, such as, e.g., above about 100 to about 115, the dentifrice does not necessarily exhibit a corresponding increase in the cleaning performance of the dentifrice. It has been challenging to formulate oral compositions that have a PCR of greater than about 80 or 90 but still have an RDA that is below 250, more preferably below 200.

However, in certain embodiments of the present invention, an oral composition has been achieved that has a PCR of greater than 100, while having an RDA of less than 200. In certain embodiments, the RDA is less than or equal to about 175, while still having a PCR that exceeds about 100. In certain embodiments, the RDA is less than 165. In this regard, the oral compositions of the present invention provide a superior cleaning and/or polishing efficacy, while achieving a desirably low RDA that minimizes potential damage to enamel or dentin.

The present compositions may provide cleaning and/or polishing of a tooth surface. Cleaning and/or polishing abrasives can be classified by various physical parameters. As appreciated by one of skill in the art, a single abrasive species typically performs at least some cleaning and polishing simultaneously. However, particles are generally categorized in the art by the predominant effect they have on a target oral surface.

As used herein, the term "cleaning" generally refers to the removal of contaminants, dirt, impurities, and/or extraneous matter on a target surface. For example, in the context of oral surfaces, where the surface is tooth enamel, the cleaning may remove at least some of a film or stain, such as plaque biofilm, pellicle or tartar. As used herein, "polishing" generally refers to a finishing or refining process that makes a surface smoother and/or glossier. Polishing and cleaning can also provide brightening of the surface where stain removal occurs, for example, whitening of a tooth surface.

Typically, "polishing abrasives" are considered to be relatively small particles having high hardness, where abrasives with relatively large particle sizes and low hardness are considered to be "cleaning abrasives." Further, the behavior the abrasive exhibits as it interacts with the surface indicates how well it will polish (or "lap") the surface, as a desirable polishing agent usually degrades into progressively smaller fragments as contact with the surface proceeds. The lapping behavior relates to the ease with which an abrasive breaks down to a successively finer particle sizes and is generally believed to be based on the abrasive particle's crystalline shape, lines of cleavage, and friability, for example.

Thus, an abrasive is generally categorized on a gradient extending from softer cleaning abrasives to harder polishing abrasives. In certain embodiments, a method of cleaning and/or polishing a target oral surface comprises contacting the surface with an oral composition that comprises a first abrasive and a second abrasive. The first abrasive of the composition may be a distinct species from the second abrasive, meaning that each abrasive may have a different effect on the target surface; for example, categorized in a different category on the abrasiveness gradient. Any number of abrasives can be selected in this manner. Thus, the first abrasive may be a relatively low cleaning abrasive and the second abrasive may be a relatively high cleaning abrasive. The first abrasive may be a cleaning abrasive and the second abrasive may be a polishing abrasive. It should be noted that the oral compositions optionally comprise a plurality of different abrasive species, and are not solely limited to the first and second abrasives.

Other parameters are also useful for categorizing abrasiveness of particles, including for example, hardness. Hardness can be expressed by a number of different tests known to those of skill in the art, including the Einlehner, Knoop, Vickers, and Rockwell hardness tests, as well as the Mohs scale of hardness. One particularly useful method of evaluating abrasive particles is the Einlehner harness value. Einlehner hardness value is obtained using an Einlehner At-1000 Abrader to measure the softness of the abrasive particle in the following manner: a Fourdrinier brass wire screen is weighed and exposed to the action of a suspension of the abrasive (for example, a 10% aqueous suspension of the abrasive) for a given number of revolutions. The hardness value is expressed as milligrams weight lost of the Fourdrinier wire screen per 100,000 revolutions. Thus, in certain embodiments, the oral composition comprises a first abrasive having an Einlehner hardness of greater than about 5 mg loss per 100,000 revolutions and a second abrasive having an Einlehner hardness of less than about 5 mg loss per 100,000 revolutions. In certain embodiments, the first abrasive has an Einlehner hardness of greater than about 10 mg loss per 100,000 revolutions and a second abrasive having an Einlehner hardness of less than about 10 mg loss per 100,000 revolutions.

When the first particle has an Einlehner hardness of greater than 5 mg loss per 100,000 revolutions, it may have more of a polishing function when it is contacted with an oral surface. When the second particle has an Einlehner hardness of less than about 5 mg loss per 100,000 revolutions, it may have more of a cleaning function, as it is a softer particle. In certain embodiments, the second particle has a hardness of greater than about 10 mg loss per 100,000 revolutions, and in other embodiments, greater than about 15 mg loss per 100,000 revolutions.

In certain embodiments, the first abrasive is a cleaning abrasive having a hardness of less than or equal to that of the oral surface to be treated, and the second abrasive is a polishing abrasive having a hardness of greater than or equal to that of the oral surface to be treated.

The structure of an abrasive particle may also reflect abrasiveness; a relatively low structure tends to have higher abrasiveness and a relatively high structure abrasive tends to have lower abrasiveness. Particle structure may be indicated by absorption of linseed oil or dibutyl phthalate (DBP) per 100 grams. Oil absorption values can be measured using the ASTM Rub-Out Method D281.

In certain embodiments, the first abrasive has an oil absorption structure of less than about 90 cm³/100 g, and the second abrasive has an oil absorption structure of greater than about 90 cm³/100 g. In various embodiments, a particle of the first abrasive has an absorption structure of less than about 80 cm³/100 g, less than about 70 cm³/100 g, or less than about 60 cm³/100 g. In various embodiments, a particle of the second abrasive has an oil absorption of greater than about 100 cm³/ 100 g, or greater than about 110 cm³/100 g.

Particle size can be indicative of an abrasive's cleaning and/ or polishing efficacy. In certain embodiments, the first abrasive has a first particle size, and the second abrasive has a second particle size. In certain embodiments, the first particle size is less than the second particle size. Mean particle size can be measured using a Malvern Particle Size Analyzer, Model Mastersizer S, Malvern Instruments, Inc. (Southborough, Massachusetts, USA). A helium-neon gas laser beam is projected through a transparent cell that contains the abrasive suspended in an aqueous solution. Light rays that strike the particles are scattered through angles that are inversely proportional to the particle size. The photodetector array measures the quantity of light at several predetermined angles. Electrical signals proportional to the measured light flux values are then processed by a microcomputer system, against a scatter pattern predicted from theoretical particles as defined by the refractive indices of the sample and aqueous dispersant to determine the particle size distribution of the subject abrasive.

In various embodiments, the first abrasive has a mean particle size of less than about 11 µm, or less than about 10 µm. For example, examples of suitable abrasives have mean particle sizes of about 7 µm to 11 µm. Some abrasives have particle sizes of less than about 5 µm. In various embodiments, the second abrasive has a mean particle size of greater than about 8 µm, or greater than about 10 µm. In some embodiments, the second abrasive can have a mean particle size of about 8 µm to about 14 µm.

In certain embodiments, the composition is safe for oral use with humans or other animals, and any orally or cosmetically acceptable abrasive fulfilling the requirements set forth above can be selected for an oral composition. Suitable abrasives include without limitation: silica, silicate, silicon, alumina (including calcined aluminum oxide), aluminosilicates, such as bentonite, zeolite, kaolin, and mica, siliceous or diatomaceous earth, pumice, calcium carbonate, cuttlebone, insoluble phosphates, composite resins, such as melamine resin, phenolic resin, and urea-formaldehyde resin, polycarbonate, silicon carbide, boron carbide, microcrystalline wax, microcrystalline cellulose, including combinations of colloidal microcrystalline cellulose and carboxymethylcellulose, commercially available under the trade name AVICEL® from FMC Biopolymer (Philadelphia, Pennsylvania, USA) and combinations and derivatives thereof.

As used herein, "mica" refers to any of a group of hydrous aluminum silicate minerals with plate morphology and perfect basal (micaceous) cleavage. Mica can be, for example, sheet mica, scrap mica or flake mica, as exemplified by muscovite, biotite or phlogopite type micas. Among insoluble phosphates useful as abrasives are orthophosphates, polymetaphosphates and pyrophosphates. Illustrative examples are dicalcium orthophosphate dihydrate, dicalcium phosphate dihydrate, calcium hydrogen phosphate, calcium pyrophosphate, β-calcium pyrophosphate, tricalcium phosphate, calcium metaphosphate, potassium metaphosphate, and sodium metaphosphate.

Synthetic silicas include both silica gels and precipitated silicas that are prepared by the neutralization of aqueous silicate solutions with a strong mineral acid. In the preparation of silica gel, a silica hydrogel is formed which is then typically washed to low salt content. The washed hydrogel may be milled to the desired size, or otherwise dried, ultimately to the point where its structure no longer changes as a result of shrinkage. When preparing such synthetic silicas, the objective is to obtain abrasives that provide maximal cleaning (i.e., removal of stained pellicle) with minimal damage to the tooth enamel and other oral tissue.

Abrasives comprising silica are particularly useful in certain embodiments of the present invention. There has been significant difficulty in the past in using a silica based abrasive at high concentrations within an oral composition, due to chemical and physical instability in the oral composition when provided at high concentrations, mouth-feel issues, and excessive abrasiveness reflected by high RDA values. However, silica is a particularly attractive option as an abrasive, as it efficaciously cleans and/or polishes oral surfaces. Further, it is relatively inert and compatible with other ingredients in the oral composition and is relatively inexpensive. Thus, in certain embodiments of the present invention, both the first and second abrasive each comprises silica. In some embodiments, the first abrasive is selected to be a harder and smaller abrasive, e.g., a higher cleaning and/or polishing abrasive, and the second abrasive is a typical cleaning abrasive. In certain embodiments, the oral compositions may comprise a particularly efficacious combination of silica abrasive particle species.

Useful abrasive materials for preparing oral compositions include high cleaning, low structure silica abrasives, such as those marketed under the trade designation SYLODENT® XWA or SYLODENT® 783 by Davison Chemical Division of W. R. Grace & Co. (Baltimore, Maryland, USA). SYLODENT® XWA 650 is a silica hydrogel composed of particles of colloidal silica.

Exemplary silica hydrogels comprise colloidal particles of silica having, in various embodiments, an average particle size of about 3 µm to about 12 µm, or about 5 µm to about 10 µm, with a pH of about 4 to about 10, or about 6 to about 9 when measured as a 5% by weight slurry. The particles of the XWA 650 contain about 10 % to about 35 % by weight water, have a mean particle size of about 5 µm to about 12 µm, an Einlehner hardness of greater than or equal to about 5 to about 20 mg loss per 100,000 revolutions, an oil absorption of less than about 90 cm³/100 g, for example from between about 40 cm³/100 g to about 90 cm³/100 g. The abrasives have a Brunauer, Emmett and Teller (BET) surface area of about 100 to about 700 m²/g. XWA 650 has a brightness of 96.8 technidyne. Such abrasives are discussed in United States Patent No. 6,290,933 to Durga et al.

Another useful high cleaning silica abrasive is marketed as SYLODENT® XWA 300 and is a silica hydrogel containing about 10% to about 25% water by weight, where the mean particle size is about 2 µm to about 4 µm. The particles have BET surface are in the range of 150 to 400 m²/g of silica. The XWA 300 abrasive has an oil absorption of less than 90 cm³/100 g silica; and a pH, in a 5% w/w suspension in boiled (CO₂ free) demineralized water, equal to or greater than 8.5. Such abrasives are discussed in, e.g., United States Patent No. 5,939,051 to Santalucia et al.

In other embodiments, a high cleaning silica for the present invention comprises a silica product, where the particles are about 5 % to about 35% by weight water, having a mean particle size of about 7 µm to about 11 µm, an Einlehner hardness of about 12 to about 19 and an oil absorption value of about 50 cm³/100 g to about 65 cm³/100 g. A BET surface area is about 100 to about 700 m²/g of silica. The brightness is generally reported to be greater than about 95 technidyne. Such a silica product is commercially available as ZEODENT® 105 from J.M. Huber (Havre de Grace, Maryland, USA).

Other useful abrasives include typical cleaning silica abrasives, such as precipitated silicas having a mean particle size of up to about 20 µm, typically at about 8 to about 14 µm, with an oil absorption structure of greater than about 90 to about 110 cm³/100 g; for example, ZEODENT®115, marketed by J. M. Huber, that has a pH at 5% of the particles of about 6.5-7.5 and an Einlehner hardness of about 2 to 4 mg loss per 100,000 revolutions. The brightness of such a silica particle is greater than about 95. In certain embodiments, such cleaning abrasives comprise the second abrasive of the oral composition.

In certain embodiments, the oral composition is in the form of a dentifrice that is a clear or transparent gel. There, an abrasive of colloidal silica, such as those sold under the trademark SYLOID® as SYLOID® 72 and SYLOID® 74 or under the trademark SANTOCEL® 100 alkali metal alumina-silicate complexes may be particularly useful, since these abrasives have refractive indices close to the refractive indices of gelling agent-liquid (including water and/or humectant) systems commonly used in dentifrices.

In various embodiments, a first and a second abrasive are combined in an oral composition to provide unexpectedly superior cleaning capability, unanticipated mildness (relatively low abrasivity), and aesthetically acceptable oral care compositions. In various embodiments the ratio of the first abrasive to the second abrasive is about 1:1.6 to about 1.6:1. For example, in certain embodiments, a ratio of the first abrasive to the second abrasive is about 1:1. In certain embodiments, the respective amounts of the first abrasive and the second abrasive present in an oral composition are about 13 to about 21 % by weight of the oral composition. In some embodiments, the amount of the first abrasive is about 15% to about 19%, and the amount of the second abrasive is about 15% to about 19% by weight of the oral composition. In certain embodiments, the first abrasive is present at about 17% by weight and the second abrasive is present at about 17% by weight of the oral composition. In various embodiments, the total amount of abrasive, including the first and second abrasive, is greater than about 25%, greater than about 30%, or greater than about 35% by weight of the oral composition.

In some embodiments, an oral composition comprises a first abrasive having an Einlehner hardness of greater than about 5 mg loss per 100,000 revolutions and a second abrasive having an Einlehner hardness of less than about 5 mg loss per 100,000 revolutions. Further, the oral composition has a PCR of greater than about 100 and an RDA of less than about 200. In certain embodiments, the RDA is less than about 175.

In certain embodiments, the first abrasive has an oil of absorption of less than about 90 cm³/100g, and the second abrasive has an oil of absorption of greater than about 90 cm³/100g. In certain embodiments, the total amount of the abrasives is greater than about 30%. In various embodiments, the first abrasive and the second abrasive are present in amounts of about 13% to about 21 %, about 15% to about 19%, or at about 17% each by weight of the total composition. In various embodiments, the first and second abrasives optionally comprise silica.

In certain embodiments, an oral composition comprises a first abrasive comprising silica, having an Einlehner hardness of greater than about 5 mg loss per 100,000 revolutions, and an oil of absorption of less than about 90 cm³/100g; and a second abrasive comprising silica having an Einlehner hardness of less than about 5 mg loss per 100,000 revolutions, and an oil of absorption of greater than about 90 cm³/100g.

The oral composition further comprises a second abrasive comprising silica and having an oil of absorption of greater than about 90 cm³/100g and an Einlehner hardness of less than about 5 mg loss per 100,000 revolutions.

In other embodiments, methods of increasing the delivery of an active ingredient in an oral composition to an oral surface are provided. In various embodiments, the method comprises introducing an oral composition into an oral cavity containing the oral surface, wherein the oral composition comprises the active ingredient, a first abrasive and a second abrasive. In certain embodiments the first and second abrasives each comprise silica and each are present in an amount of about 13% to about 21 % by weight. The oral composition has a PCR of greater than about 100 and an RDA of less than about 200. The oral composition is contacted with the oral surface. It has been unexpectedly discovered that the delivery of the active ingredient is greater for this oral composition, when compared with a comparative composition that has an abrasive comprising silica but has a PCR of less than about 100, as will be discussed in more detail below.

In particular embodiments, the active ingredient comprises a non-ionic antibacterial ingredient, such as a halogenated diphenyl ether like triclosan, which will be discussed in more detail below. In some embodiments, the first abrasive has an oil of absorption of less than about 90 cm³/100g and an Einlehner hardness of greater than about 5 mg loss per 100,000 revolutions, and the second abrasive has an oil of absorption of greater than about 90 cm³/100g and an Einlehner hardness of less than about 5 mg loss per 100,000 revolutions. Further, the oral composition may comprise an efficacy enhancing copolymer, such as for example, a copolymer of polyvinyl methyl ether and maleic anhydride, such as the commercially available GANTREZ® products sold by ISP Corporation of Wayne, N.J., and/or an anti-caries active agent, which will be discussed in more detail below.

The oral compositions may further comprise an orally acceptable carrier. Conventional ingredients that can be used to form the carriers for oral care compositions are known to the skilled artisan. The specific composition of the carrier depends on the intended use of the composition. The carrier can be a liquid, semi-solid, or solid phase. Oral compositions can be in the form of a dentifrice (including toothpastes, toothpowders, and prophylaxis pastes), confectionaries (including gums, beads and chews), film, paint-on gels, or any other form known to one of skill in the art where abrasives are employed. Selection of specific carrier components is dependent on the desired product form.

In certain embodiments, the oral composition is in the form of a dentifrice, and the exemplary carrier is substantially semi-solid or solid. The carrier can be aqueous, i.e., comprising about 5% to about 95% water. In other embodiments, the carrier is substantially non-aqueous. The carrier optionally comprises, for example, oral care active ingredients, surface active agents, such as surfactants, emulsifiers, and foam modulators, viscosity modifiers and thickeners, humectants, diluents, fillers, additional pH modifying agents, colorants, preservatives, solvents, and combinations thereof. It is understood that while general attributes of each of the above categories of materials may differ; there may be some common attributes and any given material may serve multiple purposes within two or more of such categories of materials. As recognized by one of skill in the art, the oral compositions optionally include other materials in addition to those components previously described, including for example, emollients, moisturizers, mouth feel agents and the like. Examples of suitable carriers for oral compositions are discussed in United States Patent Nos. 6,669,929 to Boyd et al., 6,379,654 to Gebreselassie et al., and 4,894,220 to Nabi et al.

The oral care active ingredients include for example, anti-bacterial active agents, anti-tartar agents, anti-caries agents, anti-inflammatory agents, anti-sensitivity agents, enzymes, nutrients, and the like. Actives useful herein are optionally present in the compositions of the present invention in safe and effective amounts that are sufficient to have the desired therapeutic or prophylactic effect in the human or lower animal subject to whom the active is administered, without undue adverse side effects (such as toxicity, irritation, or allergic response), commensurate with a reasonable risk/benefit ratio when used in the manner of this invention. The specific safe and effective amount of the active will vary with such factors as the particular condition being treated, the physical condition of the subject, the nature of concurrent therapy (if any), the specific active used, the specific dosage form, the carrier employed, and the desired dosage regimen. Active ingredients useful for treating such conditions include those discussed in United States Patent Publication 2003/0206874 to Doyle et al. Actives among those useful herein are also discussed in United States Patent 6,290,933 to Durga et al. and United States Patent 6,685,921 to Lawlor.

Any suitable fluoride ion source can be present in the oral composition, such as those discussed in United States Patent No. 5,080,887 to Gaffar et al. Sources of fluoride ions, acid phosphatases, and pyrophosphatase enzyme inhibitors, are well known in the art as anti-caries agents. A fluoride ion source may be slightly soluble in water or may be fully water-soluble. It is characterized by its ability to release fluoride ions in water and by freedom from undesired reaction with other compounds of the oral preparation. Examples of such sources are inorganic metal and/or ammonium fluoride salts and compounds, such as, for example: sodium fluoride, potassium fluoride, ammonium fluoride, calcium fluoride; a copper fluoride, such as cuprous fluoride; zinc fluoride, barium fluoride; sodium silicafluoride, ammonium fluorosilicate, sodium fluorozirconate; and sodium monofluorophosphate, aluminum mono- and di-fluorophosphate, and fluorinated sodium calcium pyrophosphate. The fluoride source can also be an amine fluoride, such as olaflur (N'octadecyltrimethylendiamine-N,N,N'-tris(2-ethanol)-dihydrofluoride).

The amount of fluoride-providing source is dependent to some extent upon the type of source, its solubility, and the type or oral preparation, but it will be present in a non-toxic amount, generally about 0.001% to about 3.0% in the preparation. In a dentifrice preparation, e.g., dental gel, toothpaste (including cream), toothpowder, or dental tablet, an amount of such source which releases up to about 5,000 ppm of F- ion by weight of the preparation is considered satisfactory. Any suitable minimum amount of such source may be used, but in various embodiments is an amount sufficient to release about 300 to 2,000 ppm, about 500 to about 1,800 ppm or about 800 to about 1,500 ppm of fluoride ion.

The oral composition optionally comprises an anti-calculus composition, such as, for example, one or more of the anti-calculus compositions discussed in United States Patent No. 5,292,526 to Gaffar, et al. In various embodiments, the anti-calculus composition includes one or more polyphosphates. The anti-calculus composition can include at least one wholly or partially neutralized alkali metal or ammonium tripolyphosphate or hexametaphosphate salt present in the oral composition at an effective anti-calculus amount. The anti-calculus active can also include at least one water soluble, linear, molecularly dehydrated polyphosphate salt effective in an anticalculus amount. The anti-calculus active can also include a mixture of potassium and sodium salts, at least one of which is present in an effective anti-calculus amount as a polyphosphate anti-calculus agent. The anti-calculus active agent can also contain an effective anticalculus amount of linear molecularly dehydrated polyphosphate salt anti-calculus agent present in a mixture of sodium and potassium salts. The ratio of potassium to sodium in the composition can be up to less than 3:1. The polyphosphate can be present in the oral composition in various amounts, such as an amount wherein the weight ratio of polyphosphate ion to anti-bacterial agent ranges from in excess of 0.72:1 to less than 4:1, or wherein the weight ratio of the anti-bacterial enhancing agent to the polyphosphate ion ranges from about 1:6 to about 2.7:1, or wherein the weight ratio of the anti-bacterial enhancing agent to the polyphosphate ranges from about 1:6 to about 2.7:1. Other useful anticalculus agents include polycarboxylate polymers and polyvinyl methyl ether/maleic anhydride (PVM/MA) copolymers, such as GANTREZ®.

Another active agent useful in dentifrice compositions of the present invention are antibacterial agents, which may be present in amounts of about 0.001 to about 3.0% by weight of the oral composition. A non-limiting list of useful additional oral care compounds includes non-ionic antibacterial agents, including phenolic and bisphenolic compounds, such as, halogenated diphenyl ethers, including triclosan (2,4,4'-trichloro-2'-hydroxy-diphenylether, triclocarban (3,4,4-trichlorocarbanilide), as well as 2-phenoxyethanol, benzoate esters, and carbanilides. A halogenated diphenyl ether, such as triclosan, can be present in an amount of about 0.3 % by weight of the oral composition, for example.

Suitable surface active agents are those that are reasonably stable throughout a wide pH range. These compounds are well known in the art, and include non-soap anionic (*e.g.*, sodium lauryl sulfate (SLS), N-myristoyl, and N-palmitoyl sarcosine), nonionic (*e.g.*, Polysorbate 20 (polyoxyethylene 20 sorbitan monolaurate, TWEEN^{®} 20) and Polysorbate 80 (polyoxyethylene 20 sorbitan mono-oleate, TWEEN^{®} 80), Poloxamer 407, available under the trade name PLURONIC^{®} F127 from BASF Corporation), cationic, zwitterionic (e.g., cocamidopropyl betaine and lauramido propyl betaine), and amphoteric organic synthetic detergents. Examples of suitable surface active agents for use in oral compositions are discussed in, for example, United States Patent Nos. 4,894,220 to Nabi et al., 6,555,094 to Glandorf et al., and 6,706,256 to Lawlor, *inter alia.* In various embodiments, one or more surface active agents are present in the oral composition of the present invention in the range of about 0.001% to about 5%, or about 0.5% to about 2.5%.

Optional thickeners for use in oral compositions include natural and synthetic gums and colloids, such as carrageenan (Irish moss), xanthan gum, sodium carboxymethyl cellulose, starch, polyvinylpyrrolidone, hydroxyethylpropyl cellulose, hydroxybutyl methyl cellulose, hydroxypropylmethyl cellulose, and hydroxyethyl cellulose. Inorganic thickeners include amorphous silica compounds which function as thickening agents and include colloidal silicas compounds available under trademarks such as Cab-o-sil fumed silica manufactured by Cabot Corporation and distributed by Lenape Chemical (Bound Brook, N.J, USA); ZEODENT® 165 from J.M. Huber Chemicals, and Sylox 15, also known as SYLODENT® 15, available from Davison Chemical Division of W.R. Grace Corp. In various embodiments, the thickening agent is present in the dentifrice composition in amounts of about 0.1 to about 10 % by weight, or about 0.5 to about 4% by weight.

The orally-acceptable dentifrice carrier vehicle used to prepare the dentifrice composition optionally comprises a humectant. The humectant can be glycerin, sorbitol, and xylitol, propylene glycol of molecular weight in the range of about 200 to about 1,000; or other humectants and mixtures thereof. The humectant concentration typically totals about 5 to about 70% by weight of the oral composition. Water is typically present in an amount of at least about 10% by weight, and generally about 25 to 70% by weight of the oral composition.

Synthetic anionic linear polycarboxylates are efficacy enhancing agents for optional use in oral compositions having certain active ingredients, including antibacterial, anti-tartar or other active agents within the oral composition. Such anionic polycarboxylates are generally employed in the form of their free acids, or partially or fully neutralized water soluble alkali metal (*e.g.*, potassium and preferably sodium) or ammonium salts. The terms "synthetic" and "linear" exclude known thickening or gelling agents comprising carboxymethylcellulose and other derivatives of cellulose and natural gums, nor carbopols having reduced solubility due to cross-linkages.

Preferred copolymers are 1:4 to 4:1 copolymers of maleic anhydride or acid with another polymerizable ethylenically unsaturated monomer, preferably methyl vinyl ether (methoxyethylene) having a molecular weight (M.W.) of about 30,000 to about 1,000,000. One useful copolymer is methylvinylether/maleic anhydride. Examples of these copolymers are available from ISP Corporation under the trade name GANTREZ®, *e.g.*, AN 139 (M.W.1,100,000), AN 119 (M.W. 200,000); S-97 Pharmaceutical Grade (M.W. 1,500,000), AN 169 (M.W. 2,000,000), and AN 179 (M.W. 2,400,000); wherein the preferred copolymer is S-97 Pharmaceutical Grade (M.W. 1,500,000). When present, the anionic polycarboxylate is employed in amounts effective to achieve the desired enhancement of the efficacy of any antibacterial, antitartar or other active agent within the dentifrice composition. In various embodiments, a synthetic anionic polycarboxylate is included in the oral composition is present at about 0.001 to about 5%, or about about 0.1 to about 2.0% of the oral composition.

The oral composition of the present invention can be made by any of the methods known in the art for combining ingredients to make oral care compositions. Examples of methods that can be used are set forth in, e.g., United States Patent No. 6,403,059 to Martin et al.; Clinical Pharmacology for Dental Professionals (Mosby-Year Book, Inc., 3rd ed. 1989); Mosby's Dental Hygiene: Concepts, Cases and Competencies, (Daniel, S. and Harfst, S. eds., Elsevier Science Health Science Div. 2002); and Ernest W. Flick, Cosmetic and Toiletry Formulations, 2nd ed.

The present invention provides for methods and processes of using the oral compositions of the present invention to clean and/or polish oral surfaces. Further, the oral compositions optionally treat and inhibit oral conditions, such as oral inflammatory conditions, dental plaque, and dental calculus. The oral compositions can be applied to the subject in any suitable manner known in the art; for example, by introducing the oral composition to the subject's oral cavity using a suitable applicator or delivery device, such as a brush, dental strip, film, syringe, tape, pill, or any other applicator or delivery device known in the art. The compositions can be used in prophylactic methods and processes to promote and maintain oral health, appearance, maintain systemic health and the like. The oral compositions can be repeatedly applied to the subject over a number of days according to a particular treatment schedule to treat and/or inhibit stain, plaque, calculus or tartar formation. Instructions setting forth the treatment schedule can be provided in commercial packaging with the product, as commercially prepared and stored.

The present invention is further illustrated through the following non-limiting example(s).

### EXAMPLE I

A dentifrice composition according to the present invention, having the ingredients listed as Dentifrice 1 in Table I is prepared by the following method: Sodium saccharin, sodium benzoate, sodium monofluorophosphate, and any other salts are dispersed in water and mixed in a conventional mixer under agitation. The humectants e.g., glycerin and sorbitol, are added to the water mixture under agitation. Then organic thickeners, such as sodium carboxymethyl cellulose, carrageen and any polymers, such as GANTREZ® are added.

The resultant mixture is agitated until a homogeneous gel phase is formed. The mixture is then transferred to a high-speed vacuum mixer; where the abrasives are added. The mixture is then mixed at high speed for from 5 to 30 minutes, under vacuum of about 20 to 50 mm Hg, preferably about 30 mmHg. The flavor oil is weighed out and triclosan is then added to the flavor oil. The flavor oil and flavonoid mixture is added to the mixture. Surfactants, such as sodium lauryl sulfate (SLS) are charged into the mixer. The resultant product is a homogeneous, semi-solid, extrudable paste or gel product. The resulting dentifrice can be applied with a brush or other applicator to the oral surfaces.

Dentifrices A and B are comparative examples and are prepared in the same manner as described above for Dentifrice 1.

**Table I**

| **INGREDIENTS** | **DENTIFRICE 1 (weight %)** | **Comparative DENTIFRICE A (weight %)** | **Comparative DENTIFRICE B (weight %)** |
|---|---|---|---|
| Glycerin | 8-15 | 15-25 | 15-25 |
| Sorbitol (70% in water) | 15-25 | 15-25 | 15-25 |
| Sodium Carboxymethyl | 0.1-5 | 0.1-5 | 0.1-5 |
| GANTREZ® S-97 | 1-3 | 1-3 | 1-3 |
| Carrageenan | 0.1-1 | 0.1-1 | 0.1-1 |
| Sodium Saccharin | 0.1-1 | 0.1-1 | 0.1-1 |
| Sodium Benzoate | 0.1-3 | 0.1-3 | 0.1-3 |
| Sodium Monofluorophosphate | 0.01-1 | 0.01-1 | 0.01-1 |
| Triclosan | 0.01-3 | 0.01-3 | 0.01-3 |
| Sodium Lauryl Sulfate | 0.5-3 | 0.5-3 | 0.5-3 |
| Flavor Oil | 0.1-5 | 0.1-5 | 0.1-5 |
| SYLODENT® XWA 650 | 15-18 | 19-21 | 7-14 |
| ZEODENT® 115 | 15-20 | --- | 8-14 |
| Water | Q.S. | Q.S. | Q.S. |

### EXAMPLE II

PCR and RDA data is shown in Table II for Dentifrice 1 prepared in accordance with the present invention, as compared to Dentifrices A and B.

The RDA is determined according to the method recommended by the American Dental Association as set forth by Hefferren, Journal of Dental Research, Volume 55, Issue 4, July-August 1976, pp. 563-573, and described in the United States Patent Nos. 4,340,583, 4,420,312, and 4,421,527 all to Wason.

PCR, as described above, is an *in vitro* method used to measure the efficacy of removing tea and coffee tooth stains relative to a standard. The PCR values referred to herein are obtained by a modification of the method described in "In Vitro Removal of Stain with Dentifrice", G. K. Stookey, et al J. Dental Research, 61, 123-9 (1982). The modification of the PCR method used herein is described in United States Patent Nos. 5,658,553 and 5,651,958 both to Rice. In this modification, a clear pellicle material is applied to a bovine tooth first, which is then stained with a combination of the pellicle material and tea, coffee and FeCl₃ whereas in the original method described by Stookey et al. both pellicle and stain are applied simultaneously.

**Table II**

| Example | PCR | % Increase of PCR | RDA | % Increase of |
|---|---|---|---|---|
| Dentifrice 1 | 108 | 27 | 163 | 18 |
| Dentifrice A | 98 | 15 | 180 | 30 |
| Dentifrice B | 85 | --- | 138 | --- |

Dentifrice B is a typical oral composition formulation having a combination of high cleaning silica (XWA 650) and regular cleaning silica (ZEODENT® 115) at 10% by weight, respectively. Dentifrice B is the control having a PCR of about 85 and an RDA of 138. As can be observed, in Dentifrice A the high cleaning silica (XWA 650) is doubled to 20% while removing the regular cleaning silica (ZEODENT® 115). For Dentifrice A, the RDA increases to 180, but the PCR only increases to about 98. In comparison, Dentifrice 1 has 17% of both the high cleaning silica (XWA 650) and the regular cleaning silica (ZEODENT® 115), but the RDA is only 163, while the PCR exceeds 100 (about 108). Thus, the combination of abrasives in Dentifrice 1 provides an unexpectedly low RDA (with only an 18% increase over Dentifrice B) while achieving a 27% increase in PCR over Dentifrice B. Comparatively, Dentifrice A has an RDA increase of over 30% and a PCR increase of only 15%, when compared to Dentifrice B.

It is discovered, therefore, that desirably high PCR values for a dentifrice composition can be achieved, while the RDA values are significantly less than would be expected.

### EXAMPLE III

An *in vitro* polishing analysis is conducted with Dentifrice 1, Dentifrice B, a dentifrice prepared in accordance with Table III below and designated Dentifrice C, and a commercially available whitening dentifrice designated Dentifrice D. Dentifrice C is prepared in the same manner as described above for Dentifrice 1 in Example I, and contains 20% by weight of regular cleaning silica, ZEODENT® 115. Dentifrice D is commercially available as CREST® Dual Action Whitening Toothpaste from Procter and Gamble of Cincinnati, OH, and is believed to contain the ingredients sodium fluoride at 0.243 %, glycerin, hydrated silica, water, sorbitol, sodium hexametaphosphate (for tartar control/stain prevention), propylene glycol, flavor, PEG-12, cocamidopropyl betaine, SLS, CARBOMER® 956, sodium saccharin, Poloxamer 407, polyethylene oxide, xanthan gum, sodium hydroxide, cellulose gum, sodium hydroxide, titanium dioxide, and Blue 1 and Yellow 5 colorings.

**Table III**

| **INGREDIENTS** | **DENTIFRICE C (weight %)** |
|---|---|
| Glycerin | 15-30 |
| Sorbitol (70% in water) | 15-30 |
| Sodium Carboxymethyl | 0.5-5 |
| GANTREZ® S-97 | 1-5 |
| Carrageenan | 0.1-2 |
| Sodium Saccharin | 0.1-2 |
| Sodium Benzoate | 0.1-2 |
| Sodium Monofluorophosphate | 0.05-1 |
| Triclosan | 0.1-2 |
| Sodium Lauryl Sulfate | 1-4 |
| Flavor Oil | 0.05-5 |
| ZEODENT® 115 | 15-25 |
| Water | Q.S. |

Table IV shows the *in vitro* mean polishing analysis of these dentifrices. The maximum reflectance of the dulled tooth surface is determined by means of a reflectometer especially adapted to detect the changes in the degree of polish of the enamel surface. The reflectometer is constructed so that the enamel is exposed to a beam of polarized light, the amount of light reflected from the enamel surface was determined by a photoelectric cell which in turn activated a galvanometer. The smoother the enamel surface, the smaller the amount of diffused and absorbed light and, hence, the higher the galvanometer reading.

After the maximum reflectance of the dulled tooth is determined, the tooth is brushed with the dentifrice to be tested. The enamel surface is then rinsed with water to remove any residual particles of the cleaning and polishing agent, and the reflectance of the enamel surface is again measured with the tooth located in exactly the same position as that used to obtain the "dull" reading. The absolute change in the amount of reflectance between the dull and polished enamel surfaces is taken as a measure of the degree of polishing imparted by the treatment. The total brushing period is about 30,000 strokes. The results in Table IV are expressed in terms of the mean increment in the luster of the enamel specimens.

Dentifrice C is a conventional dentifrice having 20 % of a regular cleaning silica abrasive, and is selected as the Control for calculating the percentage increase in mean polish increment. It is observed that Dentifrice 1 demonstrates the highest increase in mean polishing increment.

**TABLE IV**

| Example | Mean Polish Increment | % Increase in Mean Polish Increment |
|---|---|---|
| Dentifrice 1 | 3.93 | 11 |
| Dentifrice B | 3.79 | 7 |
| Dentifrice C | 3.54 | --- |
| Dentifrice D | 3.59 | 1 |

### EXAMPLE IV

The average uptake of triclosan on a hydroxyapatite disk is set forth in Table V. Dentifrices 1, B, and C are compared for triclosan uptake levels. The uptake of an active ingredient triclosan is assessed using disks of hydroxyapatite (HAP) available from Clarkson Chromatography Products, Inc., which are saliva coated (SCHAP), as an *in vitro* model for human teeth. This *in vitro* model has been found to be correlated to *in vivo* delivery of and retention of antibacterial agents on oral surfaces.

To determine the delivery and retention of triclosan to a SCHAP disk from a dentifrice containing triclosan and an anionic copolymer of the present invention, namely AMPS terpolymer, and VCAP copolymer, SCHAP disks were treated with dentifrice slurry compositions identified in Table I above. The amounts of dentifrice slurry used to contact the disks simulated in vivo surface to volume ratios found in the mouth, to simulate, in part, brushing condition. After incubation for 30 minutes at 37°C, the SCHAP disks were removed from the dentifrice slurry and washed three times with water. The uptake absorption of triclosan, on SCHAP disks, from Dentifrices 1, B, and C are set forth in Table V, below.

**TABLE V**

| Example | Average Triclosan Uptake for a HAP disk |
|---|---|
| Dentifrice 1 | 80 ± 2.6 |
| Dentifrice B | 65 ± 3.2 |
| Dentifrice C | 65 ± 3.2 |

The results in Table V show that delivery and retention of triclosan to SCHAP disks from Dentifrice 1 is substantially greater than for either of comparative examples of Dentifrices B and C. Thus, these compositions provide methods of increasing delivery of an active ingredient in an oral composition to an oral surface.

### EXAMPLE V

Various samples (A-E) were prepared and tested in accordance with the present invention. Sample A represents non high-cleaning silica. Samples B-E represent various high cleaning silica compositions. All were tested with an Einlehner Abrasian Analysis. The samples were dispersed at 10& W/V (100g diluted to a volume of 1000 mL) and run in duplicate on an Einlehner AT 1000. Results are shown in Table 6 Below:

**TABLE VI**

| **Sample** | **Program/# of Revolutions** | **Milligrans Loss** | | **Correcetd (mg loss at 174,000 revs)** |
|---|---|---|---|---|
| | | **Run 1** | **Run 2** | |
| A | 5/174,000 | 5.9 | 4 | 5.0 |
| B | 5/174,000 | 15.5 | 16.9 | 16.2 |
| C | 5/174,000 | 18.9 | 20.8 | 19.8 |
| D | 5/174,000 | 21.1 | 19.5 | 22.1 |
| E | 5/174,000 | 22.2 | 22.0 | |

## Claims

1. An oral composition comprising:
a first abrasive comprising silica, having an Einlehner hardness of greater than about 5 mg loss per 100,000 revolutions and an oil of absorption of less than about 90 cm³/100g; and
a second abrasive comprising silica, having an Einlehner hardness of less than about 5 mg loss per 100,000 revolutions and an oil of absorption of greater than about 90 cm³/100g;
wherein the first abrasive is present in an amount of about 13% to about 21% by weight and the second abrasive is present in an amount of about 13% to about 21 % by weight of the composition.

2. The composition according to claim 1, wherein the first abrasive is present in an amount of about 15% to about 19% by weight and the second abrasive is present in an amount of about 15% to about 19% by weight of the oral composition.

3. The composition according to claim 2, wherein the first abrasive is present in an amount of about 17 % by weight and the second abrasive is present in an amount of about 17% by weight of the oral composition.

4. The composition according to claim 1, wherein the oral composition has a pellicle cleaning ratio of greater than about 100 and a radioactive dentin abrasion of less than about 200.

5. The composition according to claim 4, wherein the radioactive dentin abrasion is less than about 175.

6. The composition according to claim 1, wherein a total amount of the first and second abrasives in the oral composition is greater than about 30%.

7. The composition according to claim 1, wherein the first abrasive has a mean particle size of less than about 11 µm, and the second abrasive has a mean particle size of greater than about 8 µm.

8. An oral composition comprising:
a first abrasive comprising silica and having an oil of absorption of less than about 90 cm³/100g and an Einlehner hardness of greater than about 5 mg loss per 100,000 revolutions; and
a second abrasive comprising silica and having an oil of absorption of greater than about 90 cm³/100g and an Einlehner hardness of less than about 5 mg loss per 100,000 revolutions;
wherein a ratio of the first abrasive to the second abrasive is about 1:1.6 to about 1.6:1, and a total amount of the first and second abrasives present in the oral composition is greater than about 25% by weight of the composition; and wherein the oral composition has a pellicle cleaning ratio of greater than about 100 and a radioactive dentin abrasion of less than about 200.

9. The composition according to claim 8, wherein the ratio of the first abrasive to the second abrasive is about 1:1.

10. The composition according to claim 8, wherein the first abrasive is present in an amount of about 17 % by weight and the second abrasive is present in an amount of about 17% by weight in the oral composition.

11. A method of increasing delivery of an active ingredient in an oral composition to an oral surface comprising:
introducing an oral composition into an oral cavity, wherein the oral composition comprises the active ingredient, a first abrasive and a second abrasive, wherein the first and second abrasives each comprise silica and each are present in an amount of about 13 % to about 21 % by weight, wherein the oral composition has a pellicle cleaning ratio of greater than about 100 and a radioactive dentin abrasion of less than about 200; and
contacting the composition with the oral surface, wherein the delivery of the active ingredient is greater than a comparative composition having an abrasive comprising silica and a pellicle cleaning ratio of less than about 100.

12. The method according to claim 11, wherein a ratio of the first abrasive to the second abrasive is about 1:1.6 to about 1.6:1.

13. The method according to claim 11, wherein the active ingredient comprises a halogenated diphenyl ether.

14. The method according to claim 11, wherein a ratio of the first abrasive to the second abrasive is about 1:1.6 to about 1.6:1.

15. The method according to claim 11, wherein the first abrasive has an oil of absorption of less than about 90 cm³/100g and an Einlehner hardness of greater than about 5 mg loss per 100,000 revolutions, and the second abrasive has an oil of absorption of greater than about 90 cm³/100g and an Einlehner hardness of less than about 5 mg loss per 100,000 revolutions.
